# EUROPEAN PATENT APPLICATION

(11) **EP 4 781 973 A1**
(43) Date of publication of application: **29.07.2026**
(21) Application number: 24868385.6
(22) Date of filing: 20.09.2024
(51) Int. Cl.: A61K 8/44, A61K 8/36, A61Q 5/02, A61Q 19/10, C11D 1/04, C11D 1/10, C11D 3/33

(54) **DETERGENT COMPOSITION, METHOD FOR PRODUCING SAME, AND DETERGENT PRODUCT CONTAINING DETERGENT COMPOSITION**

(30) Priority: 21.09.2023 JP 2023156575
(71) Applicant: AJINOMOTO CO., INC., Chuo-ku Tokyo 104-8315 (JP)
(72) Inventor: NONAKA, Takuya, Kawasaki-shi, Kanagawa 210-8681 (JP); AKIYOSHI, Yuma, Kawasaki-shi, Kanagawa 210-8681 (JP); KOSONO, Shuhei, Yokkaichi-shi, Mie 510-0885 (JP); KODA, Ryuji, Yokkaichi-shi, Mie 510-0885 (JP)
(74) Representative: Mewburn Ellis LLP
(86) International application number: PCT/JP2024/033781
(87) International publication number: WO 2025/063309

(57) **Abstract**

The present invention relates to a cleansing composition containing (A) one or more kinds selected from the group consisting of N-acyl alanine having an acyl group having 8 to 22 carbon atoms and a salt thereof, (B) one or more kinds selected from the group consisting of fatty acid having 8 to 22 carbon atoms and a salt thereof, and (C) one or more kinds selected from the group consisting of alanine and a salt thereof, and having a content ratio of the aforementioned component (A), component (B), and component (C) [(A):(B):(C)] of 100:45 to 245:10 to 150 by weight.

According to the present invention, a cleansing composition containing N-acyl neutral amino acid or a salt thereof can be provided, which is superior in foaming, foam quality, and a feeling of use, can impart a good moist feeling to the skin and hair after washing and drying, and further improves a frictional feeling after washing.

## Description

### [Technical Field]

The present invention relates to a cleansing composition containing N-long chain acyl neutral amino acid or a salt thereof, a production method thereof, and a cleaning agent containing the aforementioned cleansing composition.

### [Background Art]

N-long chain acyl neutral amino acids or salts thereof have superior surfactant action and superior bacteriostatic action, and are low irritative. Therefore, they are used as cleaning agent components of various cleansing compositions.

However, when N-long-chain acyl neutral amino acids or salts thereof are used alone as cleaning agents, problems occur in that foam volume and foam retentivity are insufficient, and the feeling of use, such as silky smoothness and moisturized feel of skin and hair after washing and drying, is not sufficiently satisfactory. Therefore, various studies have been conducted to address these problems.

For example, there have been proposed a cleansing composition containing N-long-chain acyl dipeptides, such as an N-long-chain acyl glycylglycine salt or an N-long-chain acyl β-alanyl β-alanine salt, and an N-long-chain acyl glycine salt or an N-long-chain acyl β-alanine salt at a specific ratio (Patent Literature 1), and a cleansing composition containing an N-long-chain acyl alanylalanine salt (Patent Literature 2).

The cleansing composition described in Patent Literature 1 was superior in foaming and foam retention, and showed good disappearance of foam during rinsing. However, a feeling of tightness remained after washing and a moist feeling after washing was insufficient.

Furthermore, the cleansing composition described in Patent Literature 2 was superior in foaming ability and foam retentivity, and was improved in resistant to hard water and frictional feeling during use; however, smooth feeling of hair after washing was insufficient.

Furthermore, as a cleansing composition superior in foaming and foam retention and capable of imparting a smooth feeling and a moist feeling to skin and hair after washing and drying, a cleansing composition containing an N-acyl neutral amino acid or a salt thereof, an N-long-chain acyl neutral amino acid dipeptide or a salt thereof, and an N-long-chain acyl neutral amino acid tripeptide or a salt thereof at a specific content ratio has been proposed (Patent Literature 3). Patent Literature 3 also proposes a method of mixing and heating a long-chain fatty acid, a neutral amino acid, and an alkaline substance to carry out a direct dehydration condensation reaction, as a production method that reduces the environmental burden compared to the conventional Schotten-Baumann reaction using fatty acid chlorides.

However, a new problem was found with the cleansing composition described in Patent Literature 3 that a frictional feeling occurs after washing.

Therefore, a cleansing composition containing N-acyl neutral amino acid or a salt thereof, that is superior in foaming, foam quality, and feeling of use, can impart a good moist feeling to skin and hair after washing and drying, and improves a frictional feeling after washing, has been demanded.

### [Citation List]

### [Patent Literature]

[Patent Literature 1]
   JP 5-078693 A
[Patent Literature 2]
   JP 7-188694 A
[Patent Literature 3]
   WO 2005/033255

### [Summary of Invention]

### [Technical Problem]

Therefore, the present invention aims to provide a cleansing composition containing N-acyl neutral amino acid or a salt thereof, that is superior in foaming, foam quality, and feeling of use, can impart a good moist feeling to skin and hair after washing and drying, and further improves a frictional feeling after washing.

### [Solution to Problem]

The present inventors have conducted intensive studies in an attempt to solve the above-mentioned problems and found that a cleansing composition containing (A) one or more kinds selected from the group consisting of N-acyl alanine having an acyl group having 8 to 22 carbon atoms and a salt thereof, (B) one or more kinds selected from the group consisting of fatty acid having 8 to 22 carbon atoms and a salt thereof, and (C) one or more kinds selected from the group consisting of alanine and a salt thereof, at a content ratio thereof [(A):(B):(C)] of 100:45 to 245:10 to 150 by weight improves foaming, foam quality, and feeling of use, as well as a moist feeling after washing and drying, and further, a frictional feeling after washing. They have conducted further studies and completed the present invention.

That is, the present invention relates to the following.
[1] A cleansing composition comprising (A) one or more kinds selected from the group consisting of N-acyl alanine having an acyl group having 8 to 22 carbon atoms and a salt thereof, (B) one or more kinds selected from the group consisting of fatty acid having 8 to 22 carbon atoms and a salt thereof, and (C) one or more kinds selected from the group consisting of alanine and a salt thereof, and having a content ratio of the aforementioned component (A), component (B), and component (C) [(A):(B):(C)] of 100:45 to 245:10 to 150 by weight.
[2] The cleansing composition of [1], further comprising (D) one or more kinds selected from the group consisting of N-acylalanylalanine having an acyl group having 8 to 22 carbon atoms and a salt thereof.
[3] The cleansing composition of [2], wherein a content ratio of component (D) to component (A) [(A):(D)] is 100:1 to 10 by weight.
[4] The cleansing composition of any of [1] to [3], comprising two or more kinds of N-acyl alanines having an acyl group having a different carbon number or salts thereof as the (A) one or more kinds selected from the group consisting of N-acyl alanine having an acyl group having 8 to 22 carbon atoms and a salt thereof.
[5] The cleansing composition of any of [1] to [4], comprising two or more kinds of fatty acids having a different carbon number or salts thereof as the (B) one or more kinds selected from the group consisting of fatty acid having 8 to 22 carbon atoms and a salt thereof.
[6] The cleansing composition of any of [2] to [5], comprising two or more kinds of N-acyl alanylalanines having an acyl group having a different carbon number or salts thereof as the (D) one or more kinds selected from the group consisting of N-acyl alanylalanine having an acyl group having 8 to 22 carbon atoms and a salt thereof.
[7] A method for producing a cleansing composition, comprising reacting (a) fatty acid having 8 to 22 carbon atoms, (b) alanine, and (c) one or more kinds selected from the group consisting of alkali metal hydroxide and amine at 147°C to 190°C.
[8] The production method of [7], wherein the (a) fatty acid having 8 to 22 carbon atoms is used in 0.5 moles to 1.5 moles per mole of (b) alanine.
[9] The production method of [7] or [8], wherein the (c) one or more kinds selected from the group consisting of alkali metal hydroxide and amine is used in 0.5 moles to 1.5 moles per mole of (b) alanine.
[10] The production method of any of [7] to [9], wherein the (a) fatty acid having 8 to 22 carbon atoms is a mixture of two or more kinds of fatty acids having a different carbon number.
[11] The production method of any of [7] to [10], wherein the (b) alanine is L-alanine or DL-alanine.
[12] The production method of any of [7] to [11], wherein the (c) one or more kinds selected from the group consisting of alkali metal hydroxide and amine is sodium hydroxide.
[13] A cleaning agent comprising the cleansing composition of any of [1] to [6] as a cleaning agent component.
[14] The cleaning agent of [13], having a viscosity of 500 mPa·s to 60,000 mPa·s as measured using a B type viscometer at 25°C.
[15] A cleaning agent comprising the cleansing composition of any of [1] to [6] as a cleaning aid.
[16] The cleaning agent of [15], having a viscosity of 500 mPa·s to 60,000 mPa·s as measured using a B type viscometer at 25°C.
[17] A cleaning agent comprising the cleansing composition of any of [1] to [6] as a cleaning agent component and further comprising a thickener.
[18] The cleaning agent of [17], wherein the thickener is one or more kinds selected from the group consisting of hydroxypropyl starch phosphate, fatty acid alkanolamide, glycine-type amphoteric surfactant, aminoacetic acid betaine-type amphoteric surfactant, sulfobetaine-type amphoteric surfactant, and amidoamine oxide-type amphoteric surfactant.
[19] The cleaning agent of [17] or [18], wherein a content of the thickener is 1 wt% to 5 wt% with respect to the total amount of the cleaning agent.
[20] The cleaning agent of any of [17] to [19], having a viscosity of 500 mPa·s to 400,000 mPa·s as measured using a B type viscometer at 25°C.
[21] A cleaning agent comprising the cleansing composition of any of [1] to [6] as a cleaning aid and further comprising a thickener.
[22] The cleaning agent of [21], wherein the thickener is one or more kinds selected from the group consisting of hydroxypropyl starch phosphate, fatty acid alkanolamide, glycine-type amphoteric surfactant, aminoacetic acid betaine-type amphoteric surfactant, sulfobetaine-type amphoteric surfactant, and amidoamine oxide-type amphoteric surfactant.
[23] The cleaning agent of [21] or [22], wherein a content of the thickener is 1 wt% to 5 wt% with respect to the total amount of the cleaning agent.
[24] The cleaning agent of any of [21] to [23], having a viscosity of 500 mPa·s to 400,000 mPa·s as measured using a B type viscometer at 25°C.

### [Advantageous Effects of Invention]

The present invention can provide a cleansing composition that is superior in foaming and foam quality, affords good feeling of use, imparts a moist feeling after washing and drying, and further improves a frictional feeling after washing. The aforementioned cleansing composition can be used as a cleaning agent component or a cleaning aid in cleaning agents for skin or hair.

In addition, the present invention can provide a method for producing a cleansing composition that imposes little burden on the environment, allows the reaction to proceed under relatively mild conditions, and allows the reaction mixture to be used as is as a cleansing composition.

Furthermore, the present invention can provide a cleaning agent containing the above-mentioned cleansing composition, that is superior in foaming and foam quality, can impart a good moist feeling to skin or hair after washing and drying, improves a frictional feeling after washing, and is superior in a feeling of use.

### [Description of Embodiments]

The present invention provides a cleansing composition. In the present specification, the "cleansing composition" refers to, as described below, a composition contained as a cleaning agent component or cleaning aid in a cleaning agent for skin or hair.

The cleansing composition of the present invention contains (A) one or more kinds selected from the group consisting of N-acyl alanine having an acyl group having 8 to 22 carbon atoms and a salt thereof, (B) one or more kinds selected from the group consisting of fatty acid having 8 to 22 carbon atoms and a salt thereof, and (C) one or more kinds selected from the group consisting of alanine and a salt thereof.

The alanine constituting the N-acyl alanine contained as component (A) in the cleansing composition of the present invention is a 2-aminopropanoic acid which is a neutral aliphatic amino acid. It may be any of L-form, D-form, and DL-form, and L-form and DL-form are preferred.

The acyl group in the N-acyl alanine contained as component (A) in the cleansing composition of the present invention is a long chain acyl group having 8 to 22 carbon atoms, and examples include straight-chain or branched-chain saturated acyl groups having 8 to 22 carbon atoms, such as octanoyl (capryloyl), 6-methylheptanoyl (isooctanoyl), 2-ethylhexanoyl, decanoyl (caprinoyl), dodecanoyl (lauroyl), tetradecanoyl (myristoyl), 12-methyltridecanoyl (isomyristoyl), hexadecanoyl (palmitoyl), 14-methylpentadecanoyl (isopalmitoyl), octadecanoyl (stearoyl), 16-methylheptadecanoyl (isostearoyl), eicosanoyl (arachidoyl), docosanoyl (behenoyl), and the like, straight-chain or branched-chain unsaturated acyl groups having 8 to 22 carbon atoms, such as octenoyl, 6-methylheptenoyl (isooctenoyl), decenoyl, dodecenoyl, tetradecenoyl, 9-hexadecenoyl (palmitreinoyl), 9-octadecenoyl (oleoyl), 11-octadecenoyl (vaccenoyl), cis,cis-9,12-octadecadienoyl (linoleoyl), 9,12,15-octadecatrienoyl (9,12,15-linolenoyl), 6,9,12-octadecatrienoyl (6,9,12-linolenoyl), 5,8,11,14-eicosatetraenoyl (arachidonoyl), cis-13-docosenoyl, and the like. It may be a mixture of saturated acyl group and unsaturated acyl group each derived from a fatty acid mixture mainly containing fatty acids having 8 to 18 carbon atoms, or a mixture of saturated acyl group and unsaturated acyl group each derived from fatty acid derived from a vegetable oil mainly containing fatty acids having 8 to 18 carbon atoms, such as coconut oil fatty acid acyl (cocoyl), palm oil fatty acid acyl, palm kernel oil fatty acid acyl, and the like.

In the cleansing composition of the present invention, a salt of the above-mentioned N-acyl alanine can also be used as component (A).

The salt of N-acyl alanine is not particularly limited as long as it is a pharmaceutically acceptable salt used in compositions for skin, and acid addition salts, salts with bases, and the like can be mentioned.

Specifically, salts with inorganic bases, organic bases, inorganic acids, and organic acids, salts with amino acids, and the like can be mentioned.

Examples of the salts with inorganic bases include salts with alkali metals such as lithium, sodium, potassium and the like, salts with alkaline earth metals such as magnesium, calcium and the like, ammonium salt, and the like.

Examples of the salts with organic bases include salts with alkanol amines such as monoethanolamine, diethanolamine, triethanolamine and the like, salts with heterocyclic amines such as morpholine, piperidine and the like, and the like.

Examples of the salts with inorganic acids include salts with hydrohalic acid (hydrochloric acid, hydrobromic acid, hydroiodic acid, etc.), sulfuric acid, nitric acid, phosphoric acid, and the like, and the like.

Examples of the salts with organic acids include salts with monocarboxylic acids such as formic acid, acetic acid, propanoic acid, and the like; salts with saturated dicarboxylic acids such as oxalic acid, malonic acid, succinic acid, and the like; salts with unsaturated dicarboxylic acids such as maleic acid, fumaric acid, and the like; salts with tricarboxylic acids such as citric acid and the like; salts with keto acids such as α-ketoglutaric acid and the like; and the like.

Examples of the salts with amino acid include salts with aliphatic amino acids such as glycine and the like; salts with aromatic amino acids such as tyrosine and the like; salts with basic amino acids such as lysine and the like; salts with acidic amino acids such as aspartic acid, glutamic acid and the like; and the like.

The above-mentioned salts may each be hydrate (hydrate salt), and examples of such hydrate include 1 hydrate to 6 hydrate and the like.

From the viewpoint of ease availability, ease of handling, and the like, alkali metal salts such as sodium salt, potassium salt, and the like, salts with alkanolamines such as monoethanolamine, diethanolamine, triethanolamine, and the like are preferably used as salts of N-acyl alanine, sodium salt, potassium salt, and triethanolamine salt are more preferably used, and sodium salt and potassium salt are further preferably used.

In the cleansing composition of the present invention, one kind of the above-mentioned N-acyl alanine and a salt thereof may be selected and used alone, or two or more kinds thereof may be selected and used in combination.

For the purposes of the present invention, it is preferable to select two or more kinds of N-acyl alanines having acyl groups having different carbon numbers or salts thereof and use them in combination as component (A).

As one or more kinds selected from the group consisting of N-acyl alanines having acyl groups having 8 to 22 carbon atoms and salts thereof contained as component (A) in the cleansing composition of the present invention, mixtures of N-acyl alanines having straight chain or branched chain saturated acyl groups having 8 to 22 carbon atoms or salts thereof are preferably used, and mixtures of N-acyl alanines having saturated acyl groups having 10 to 14 carbon atoms or salts thereof, for example, mixtures of N-decanoyl(caprinoyl) alanines or salts thereof, N-dodecanoyl(lauroyl) alanines or salts thereof, and N-tetradecanoyl(myristoyl) alanines or salts thereof are more preferably used. In addition, N-acyl alanines or salts thereof having acyl groups derived from fatty acid mixtures containing a large amount of saturated fatty acids having 8 to 18 carbon atoms are also preferably used, and N-acyl alanines or salts thereof having acyl groups derived from fatty acids derived from vegetable oils containing a large amount of saturated fatty acids having 8 to 18 carbon atoms, such as N-coconut oil fatty acid acyl alanine or a salt thereof, N-palm oil fatty acid acyl alanine or a salt thereof, N-palm kernel oil fatty acid acyl alanine or a salt thereof, and the like, are also more preferably used.

The above-mentioned N-acyl alanine and a salt thereof can be produced and used by the thermal dehydration condensation reaction of alanine, a fatty acid having 8 to 22 carbon atoms, and a basic substance in the below-mentioned production method of the cleansing composition of the present invention, or by other known production methods. In addition, commercially available products provided by various companies, including Ajinomoto Co., Inc., can be used.

Examples of the fatty acid having 8 to 22 carbon atoms contained as component (B) in the cleansing composition of the present invention include straight chain or branched chain saturated fatty acids such as octanoic acid (caprylic acid), 2-ethylhexanoic acid, nonanoic acid, decanoic acid (capric acid), dodecanoic acid (lauric acid), tetradecanoic acid (myristic acid), 12-methyltridecanoic acid (isomyristic acid), hexadecanoic acid (palmitic acid), 14-methylpentadecanoic acid (isopalmitic acid), octadecanoic acid (stearic acid), 16-methylheptadecanoic acid (isostearic acid), icosanoic acid (arachidic acid), docosanoic acid (behenic acid), and the like, and straight chain or branched chain unsaturated fatty acids such as octenoic acid, 6-methylheptenoic acid (isooctenoic acid), decenoic acid, 10-undecenoic acid, dodecenoic acid, tetradecenoic acid, 9-hexadecenoic acid (palmitoleic acid), cis-9-octadecenoic acid (oleic acid), 11-octadecenoic acid (vaccenic acid), cis,cis-9,12-octadecadienoic acid (linoleic acid), 9,12,15-octadecatrienoic acid (9,12,15-linolenic acid), 6,9,12-octadecatrienoic acid (6,9,12-linolenic acid), 5,8,11,14-eicosatetraenoic acid (arachidonic acid), cis-13-docosenoic acid (erucic acid), and the like.

In addition, a fatty acid mixture mainly containing fatty acid having 8 to 18 carbon atoms can also be used, and fatty acid derived from a vegetable oil mainly containing fatty acid having 8 to 18 carbon atoms, such as coconut oil fatty acid, palm oil fatty acid, palm kernel oil fatty acid, and the like can also be used.

In the cleansing composition of the present invention, a salt of the above-mentioned fatty acid having 8 to 22 carbon atoms can be used as component (B).

The salt of fatty acid having 8 to 22 carbon atoms is not particularly limited as long as it is a pharmaceutically acceptable salt used in compositions for skin, and salts with inorganic bases or organic bases can be mentioned.

Examples of the salts with inorganic bases include salts with alkali metals such as lithium, sodium, potassium and the like, salts with alkaline earth metals such as magnesium, calcium and the like, ammonium salt, and the like.

Examples of the salts with organic bases include salts with alkanol amines such as monoethanolamine, diethanolamine, triethanolamine and the like, salts with heterocyclic amines such as morpholine, piperidine and the like, and the like.

From the viewpoint of ease availability, ease of handling, and the like, alkali metal salts such as sodium salt, potassium salt, and the like, salts with alkanolamines such as monoethanolamine, diethanolamine, triethanolamine, and the like are preferably used as salts of fatty acid having 8 to 22 carbon atoms, sodium salt, potassium salt, and triethanolamine salt are more preferably used, and sodium salt and potassium salt are further preferably used.

In the cleansing composition of the present invention, one kind of the above-mentioned fatty acid having 8 to 22 carbon atoms and a salt thereof may be selected and used alone, or two or more kinds thereof may be selected and used in combination.

For the purposes of the present invention, it is preferable to select two or more kinds of fatty acids having different carbon numbers or salts thereof and use them in combination as component (B).

As one or more kinds selected from the group consisting of fatty acids having 8 to 22 carbon atoms and salts thereof contained as component (B) in the cleansing composition of the present invention, mixtures of straight chain or branched chain saturated fatty acids having 8 to 22 carbon atoms or salts thereof are preferably used, and mixtures of saturated fatty acids having 10 to 14 carbon atoms or salts thereof, for example, mixtures of decanoic acid (capric acid) or a salt thereof, dodecanoic acid (lauric acid) or a salt thereof, and tetradecanoic acid (myristic acid) or a salt thereof are more preferably used. In addition, mixtures of fatty acids containing large amounts of saturated fatty acids having 8 to 18 carbon atoms or salts thereof are also preferably used, and fatty acids derived from vegetable oils containing large amounts of saturated fatty acids having 8 to 18 carbon atoms or salts thereof, such as coconut oil fatty acid, palm oil fatty acid, palm kernel oil fatty acid, and the like are more preferably used.

The above-mentioned fatty acids having 8 to 22 carbon atoms and salts thereof may be extracted from plants or animals containing them, purified, and used, or may be produced by a production method known per se. Commercially available products provided by various companies may also be used.

The alanine contained as component (C) in the cleansing composition of the present invention is as described above for the alanine constituting component (A).

Salts of alanine include the same salts as those described above for the salts of N-acyl alanine, that is, salts with inorganic bases, organic bases, inorganic acids, and organic acids, salts with amino acids, and the like.

In the cleansing composition of the present invention, one kind of alanine and a salt thereof may be selected and used alone, or two or more kinds thereof may be selected and used in combination.

Alanine and salts thereof may be extracted from plants or animals containing them, purified, and used, or may be produced by a production method known per se. Commercially available products provided by various companies may also be used.

In the cleansing composition of the present invention, (A) one or more kinds selected from the group consisting of N-acyl alanine having an acyl group having 8 to 22 carbon atoms and a salt thereof, (B) one or more kinds selected from the group consisting of fatty acid having 8 to 22 carbon atoms and a salt thereof, and (C) one or more kinds selected from the group consisting of alanine and a salt thereof are contained at a content ratio of them [(A): (B):(C)] of 100:45 to 245:10 to 150 by weight, preferably at a weight ratio of 100:60 to 200:10 to 120, more preferably at a weight ratio of 100:85 to 200:10 to 80.

When the content ratio of component (B) to the content of component (A) [(B)/(A)] is 0.45 to 2.45 by weight, and the content ratio of component (C) to the content of component (A) [(C)/(A)] is 0.1 to 1.5 by weight, the foaming ability and foam quality becomes fine, the moist feeling to skin and hair after washing and drying is improved, and the frictional feeling during washing is improved.

In addition to the above-mentioned component (A) to component (C), the cleansing composition of the present invention preferably contains (D) one or more kinds selected from the group consisting of N-acylalanylalanine having an acyl group having 8 to 22 carbon atoms and a salt thereof.

The alanylalanine constituting the N-acylalanylalanine contained as component (D) in the cleansing composition of the present invention may be a dipeptide of any of L-alanine, D-alanine, and DL-alanine, and is preferably a dipeptide of L-alanine and DL-alanine.

The acyl group having 8 to 22 carbon atoms is as described above for the acyl group having 8 to 22 carbon atoms in component (A).

Salts of N-acylalanylalanine include the same salts as those mentioned above for the salt of N-acyl alanine (A).

In the cleansing composition of the present invention, one kind of N-acyl alanylalanine having acyl group having 8 to 22 carbon atoms and a salt thereof may be selected and used alone, or two or more kinds thereof may be selected and used in combination.

For the purposes of the present invention, it is preferable to select two or more kinds of N-acyl alanylalanines having acyl groups having different carbon numbers or salts thereof and use them in combination as component (D).

As one or more kinds selected from the group consisting of N-acyl alanylalanines having acyl groups having 8 to 22 carbon atoms and salts thereof contained as component (D) in the cleansing composition of the present invention, mixtures of N-acyl alanylalanines having straight chain or branched chain saturated acyl groups having 8 to 22 carbon atoms or salts thereof are preferably used, and mixtures of N-acyl alanylalanines having saturated acyl groups having 10 to 14 carbon atoms or salts thereof, for example, mixtures of N-decanoyl(caprinoyl) alanylalanines or salts thereof, N-dodecanoyl(lauroyl) alanylalanines or salts thereof, and N-tetradecanoyl(myristoyl) alanylalanines or salts thereof are more preferably used. In addition, N-acyl alanylalanines or salts thereof having acyl groups derived from fatty acid mixtures containing a large amount of saturated fatty acids having 8 to 18 carbon atoms are also preferably used, and N-acyl alanylalanines or salts thereof having acyl groups derived from fatty acids derived from vegetable oils containing a large amount of saturated fatty acids having 8 to 18 carbon atoms, such as N-coconut oil fatty acid acyl alanylalanine or a salt thereof, N-palm oil fatty acid acyl alanylalanine or a salt thereof, N-palm kernel oil fatty acid acyl alanylalanine or a salt thereof, and the like, are also more preferably used.

N-acylalanylalanine and a salt thereof may be produced by a production method known per se and be used, or may be produced by a thermal dehydration condensation reaction of a fatty acid having 8 to 22 carbon atoms, alanine, and a basic substance in the production method of the cleansing composition of the present invention described below.

The content of (D) one or more kinds selected from the group consisting of N-acyl alanylalanine having an acyl group having 8 to 22 carbon atoms and a salt thereof in the cleansing composition of the present invention is preferably 100:1 to 10, more preferably 100:1 to 3, in a weight ratio relative to component (A) [(A): (D)] .

By containing component (D), a frictional feeling and texture during washing hair with the cleansing composition is improved.

The cleansing composition of the present invention can contain general additives such as solvents such as water and the like, thickeners, pH adjusters, chelating agents, preservative, and the like, in addition to the above-mentioned components (A) to (D), as long as the features of the present invention are not impaired.

The cleansing composition of the present invention can be prepared by mixing the above-mentioned components (A) to (C) or the above-mentioned components (A) to (D), adding a solvent such as water and the like and an additive as necessary, and homogeneously mixing them.

In addition, a reaction product obtained by the below-mentioned production method of the cleansing composition of the present invention can also be used as it is as the cleansing composition of the present invention.

The cleansing composition of the present invention can be provided in various forms, such as liquid, paste, gel, solid, and the like.

The cleansing composition of the present invention is superior in foaming and foam quality, affords a good feeling of use, can impart a moist feeling after washing and drying, and further improves a frictional feeling after washing. Therefore, it can be used favorably as a cleaning agent component in cleaning agents for skin or hair, and can also be used favorably as a cleaning aid.

Also, the present invention provides a production method of a cleansing composition (hereinafter also to be referred to as "the production method of the present invention" in the present specification).

The production method of the present invention includes reacting (a) fatty acid having 8 to 22 carbon atoms, (b) alanine, and (c) one or more kinds selected from the group consisting of alkali metal hydroxide and amine at 147°C to 190°C.

The (a) fatty acid having 8 to 22 carbon atoms used in the production method of the present invention is as described above for the fatty acid having 8 to 22 carbon atoms contained as component (B) in the cleansing composition of the present invention.

In the production method of the present invention, it is preferable to select and mix two or more kinds of fatty acids having different carbon chain lengths and use same as (a) fatty acid having 8 to 22 carbon atoms, and a mixture of straight chain or branched chain saturated fatty acids having 8 to 22 carbon atoms is more preferably used. Particularly, mixtures of fatty acids having 10 to 14 carbon atoms, for example, mixtures of decanoic acid (capric acid), dodecanoic acid (lauric acid), and tetradecanoic acid (myristic acid), and fatty acids derived from vegetable oils containing large amounts of fatty acids having 8 to 18 carbon atoms, such as coconut oil fatty acid, palm oil fatty acid, palm kernel oil fatty acid, and the like are more preferably used.

The (b) alanine used in the production method of the present invention is as described above for the alanine constituting the N-acyl alanine contained as component (A) in the cleansing composition of the present invention.

In the production method of the present invention, L-alanine and DL-alanine are preferably used as (b) alanine.

In the production method of the present invention, (c) one or more kinds selected from the group consisting of alkali metal hydroxide and amine is used as a basic substance.

Examples of the alkali metal hydroxide include lithium hydroxide, sodium hydroxide, potassium hydroxide, and the like, and examples of the amine include monoethanolamine, diethanolamine, triethanolamine, and the like.

From the viewpoint of the progress and efficiency and the like of the condensation reaction between a fatty acid having 8 to 22 carbon atoms and alanine, alkali metal hydroxides are preferably used as the basic substance, sodium hydroxide and potassium hydroxide are more preferably used, and sodium hydroxide is further preferably used.

In the production method of the present invention, (a) fatty acid having 8 to 22 carbon atoms is preferably used in an amount of 0.5 to 1.5 moles, more preferably 0.7 to 1.2 moles, further preferably 0.8 to 1.0 moles, particularly preferably 0.82 to 0.89 moles, per mole of (b) alanine.

In the production method of the present invention, (c) one or more kinds selected from the group consisting of alkali metal hydroxide and amine is preferably used in an amount of 0.5 moles to 1.5 moles, more preferably 0.8 moles to 1.3 moles, per mole of (b) alanine.

In the production method of the present invention, (a) fatty acid having 8 to 22 carbon atoms, (b) alanine, and (c) one or more kinds selected from the group consisting of alkali metal hydroxide and amine are mixed and reacted at 147°C to 190°C to perform thermal dehydration condensation of (a) fatty acid having 8 to 22 carbon atoms and (b) alanine.

The above-mentioned thermal dehydration condensation reaction is preferably performed at 160°C to 180°C, more preferably 160°C to 170°C.

In addition, the above-mentioned thermal dehydration condensation reaction is preferably performed at the above-mentioned temperature for 15 hr to 30 hr, more preferably for 18 hr to 28 hr.

In the production method of the present invention, the above-mentioned thermal dehydration condensation reaction is preferably performed in an inert gas atmosphere such as nitrogen, helium, and the like to prevent coloration of the reaction mixture due to heating.

In addition, in the production method of the present invention, it is preferable to allow the reaction to proceed while removing water generated during the above-mentioned reaction, i.e., water generated by neutralization with a basic substance and water generated by the condensation reaction. Water generated during the reaction can be removed together with an inert gas stream or by reducing the pressure.

By the production method of the present invention, a reaction mixture containing N-acyl alanine having an acyl group having 8 to 22 carbon atoms or a salt thereof which is a resultant product of the above-mentioned thermal dehydration condensation reaction, an unreacted fatty acid having 8 to 22 carbon atoms or a salt thereof, and unacylated alanine or a salt thereof is obtained. In the aforementioned reaction mixture, N-acylalanine salt and fatty acid salt take the form of an alkali metal salt or an amine salt, depending on the basic substance used.

Using (a) fatty acid having 8 to 22 carbon atoms, (b) alanine, and (c) one or more kinds selected from the group consisting of alkali metal hydroxide and amine at the above-mentioned amount ratio, the reaction is performed under the above-mentioned reaction conditions, whereby a reaction mixture containing N-acyl alanine having an acyl group having 8 to 22 carbon atoms or a salt thereof, fatty acid having 8 to 22 carbon atoms or a salt thereof, and alanine or a salt thereof at the above-mentioned weight ratio of 100:45 to 245:10 to 150 can be obtained. Therefore, the aforementioned reaction mixture can be used as it is as the cleansing composition of the present invention.

The above-mentioned reaction mixture is neutralized with a mineral acid such as hydrochloric acid, sulfuric acid, and the like to convert the N-acyl alanine salt and the fatty acid salt into free N-acyl alanine and fatty acid, respectively. Then, it is neutralized with an appropriate base to obtain the desired N-acyl alanine salt and fatty acid salt.

Furthermore, in the production method of the present invention, a reaction mixture containing N-acyl alanylalanine having an acyl group having 8 to 22 carbon atoms or a salt thereof can be obtained depending on the ratio of the amounts of use of (a) fatty acid having 8 to 22 carbon atoms, (b) alanine, and (c) one or more kinds selected from the group consisting of alkali metal hydroxide and amine, and reaction conditions.

The production method of the present invention imposes little burden on the environment, allows the reaction to proceed under relatively mild conditions, and the obtained reaction mixture can be used as is as a cleansing composition.

Furthermore, the present invention provides a cleaning agent. In the present specification, the "cleaning agent" refers to a composition used when washing skin and hair.

The cleaning agent of the present invention contains the above-mentioned cleansing composition of the present invention. In the cleaning agent of the present invention, the above-mentioned cleansing composition of the present invention can be contained as a cleaning agent component or cleaning aid. In the present specification, the "cleaning agent component" refers to a component used for the purpose of washing skin and hair, and the "cleaning aid" refers to a component added to enhance the washing power of the cleaning agent component.

When the cleansing composition of the present invention is contained as a cleaning agent component, the content thereof in the cleaning agent of the present invention is preferably 1 wt% to 50 wt%, more preferably 1 wt% to 30 wt%, further preferably 10 wt% to 30 wt%, as the total amount of (A) one or more kinds selected from the group consisting of N-acyl alanine having an acyl group having 8 to 22 carbon atoms and a salt thereof, (B) one or more kinds selected from the group consisting of fatty acid having 8 to 22 carbon atoms and a salt thereof, and (C) one or more kinds selected from the group consisting of alanine and a salt thereof [(A)+(B)+(C)].

When the cleansing composition of the present invention is contained as a cleaning aid, the content thereof in the cleaning agent of the present invention is preferably 0.5 wt% to 10 wt%, more preferably 1 wt% to 7 wt%, as the total amount of (A) one or more kinds selected from the group consisting of N-acyl alanine having an acyl group having 8 to 22 carbon atoms and a salt thereof, (B) one or more kinds selected from the group consisting of fatty acid having 8 to 22 carbon atoms and a salt thereof, and (C) one or more kinds selected from the group consisting of alanine and a salt thereof [(A)+(B)+(C)].

When the cleansing composition of the present invention contains (D) one or more kinds selected from the group consisting of N-acyl alanylalanine having an acyl group having 8 to 22 carbon atoms and a salt thereof, the content of the cleansing composition of the present invention when contained as a cleaning agent component in the cleaning agent of the present invention is preferably 1 wt% to 50 wt%, more preferably 1 wt% to 30 wt%, further preferably 10 wt% to 30 wt%, as the total amount of (A) one or more kinds selected from the group consisting of N-acyl alanine having an acyl group having 8 to 22 carbon atoms and a salt thereof, (B) one or more kinds selected from the group consisting of fatty acid having 8 to 22 carbon atoms and a salt thereof, (C) one or more kinds selected from the group consisting of alanine and a salt thereof, and (D) one or more kinds selected from the group consisting of N-acyl alanylalanine having an acyl group having 8 to 22 carbon atoms and a salt thereof [(A)+(B)+(C)+(D)].

In addition, the content of the cleansing composition of the present invention when contained as a cleaning aid in the cleaning agent of the present invention is preferably 0.5 wt% to 10 wt%, more preferably 1 wt% to 7 wt%, as the total amount of (A) one or more kinds selected from the group consisting of N-acyl alanine having an acyl group having 8 to 22 carbon atoms and a salt thereof, (B) one or more kinds selected from the group consisting of fatty acid having 8 to 22 carbon atoms and a salt thereof, (C) one or more kinds selected from the group consisting of alanine and a salt thereof, and (D) one or more kinds selected from the group consisting of N-acyl alanylalanine having an acyl group having 8 to 22 carbon atoms and a salt thereof [(A)+(B)+(C)+(D)].

To the cleaning agent of the present invention, general additives such as solvents (water, ethanol, etc.), various surfactants (anionic surfactants other than the above-mentioned component (A), component (B), and component (D), cationic surfactants, amphoteric surfactants, and nonionic surfactants), oils, emollients, moisturizers, thickeners, hair conditioning agents, anti-inflammatory agents, ultraviolet absorbers, preservatives, antioxidants, chelating agents, pH adjusters, colorants, fragrances, and the like may be added as necessary, as long as the features of the present invention are not impaired.

The cleaning agent of the present invention can be provided in the forms of liquid, milk, gel, paste, cream, powder, solid, or the like, and can be provided as facial cleansers such as facial cleansing foam, facial cleansing powder, cleansing lotion, cleansing cream, and the like; body cleansers such as hand soap, body soap, bath soap, and the like; hair cleansers such as shampoo, rinse-in shampoo, and the like; and the like.

The cleaning agent of the present invention can be produced according to a general formulation method depending on the form thereof. For example, a liquid cleaning agent can be produced by adding the cleansing composition of the present invention and, where necessary, general additives to a solvent such as water and the like and blending them to form a homogeneous mixture. Milky or creamy cleaning agent can be produced by adding and dissolving the cleansing composition of the present invention and a hydrophilic additive where necessary in a solvent such as water and the like, and then adding a lipophilic additive such as oil to be added where necessary, after mixing and dissolution thereof, to the mixture and emulsifying the mixture.

From the viewpoint of usability, handling property, and the like, the cleaning agent of the present invention preferably has a moderate viscosity. For example, the viscosity is preferably 500 mPa·s to 60,000 mPa·s, more preferably 1,000 mPa·s to 60,000 mPa·s, further preferably 2,000 mPa·s to 60,000 mPa·s, as measured using a B type viscometer at 25°C.

In the cleaning agent of the present invention, the above-mentioned viscosity can be imparted by containing the cleansing composition of the present invention as a cleaning agent component or cleaning aid, or by further containing a thickener.

In addition, from the viewpoint of low irritation to the skin, the cleaning agent of the present invention is preferably weakly acidic to weakly basic, and the pH at 25°C is preferably 5 to 8.5, more preferably 5 to 7.5. The pH of the cleaning agent of the present invention can be measured as it is by the glass electrode method when it is a liquid cleaning agent, or after dissolving or suspending the cleaning agent in water when it is a solid cleaning agent.

In the cleaning agent of the present invention, the pH of the cleaning agent can be set to fall within the above-mentioned range by containing the cleansing composition of the present invention as a cleaning agent component or cleaning aid, or by further containing a pH adjuster.

In one embodiment of the present invention, a thickener can be added to the cleansing composition of the present invention to provide a more thickened cleaning agent. As the thickener, hydroxypropyl starch phosphate which is a modified starch, fatty acid alkanolamides which are nonionic surfactants, and amphoteric surfactants such as glycine-type, aminoacetic acid betaine-type, sulfobetaine-type, and amidoamine oxide-type, can be preferably used.

Hydroxypropyl starch phosphate is a hydroxypropyl ether of diester of phosphoric acid and starch.

Fatty acid alkanolamides are obtained by condensing alkanolamines with fatty acids having about 8 to 22 carbon atoms, preferably 8 to 18 carbon atoms, and examples include lauric acid monoethanolamide, lauric acid diethanolamide, myristic acid monoethanolamide, myristic acid diethanolamide, palmitic acid monoethanolamide, palmitic acid diethanolamide, stearic acid monoethanolamide, stearic acid diethanolamide, coconut oil fatty acid monoethanolamide, coconut oil fatty acid diethanolamide, and the like. From the viewpoint of thickening effect of the cleaning agent, lauric acid diethanolamide, coconut oil fatty acid monoethanolamide, coconut oil fatty acid diethanolamide, and the like are preferably used.

Examples of glycine-type amphoteric surfactants include 2-alkyl-N-carboxymethyl-N-hydroxyethylimidazolinium betaine, N-coconut oil fatty acid acyl-N-carboxymethoxyethyl-N-carboxymethylethylenediamine disodium, N-lauroyl-N'-carboxymethyl-N'-hydroxyethylethylenediamine sodium, and the like.

Examples of aminoacetic acid betaine-type amphoteric surfactants include fatty acid amidopropyl betaines such as lauric acid amidopropyl betaine, myristic acid amidopropyl betaine, coconut oil fatty acid amidopropyl betaine, palm kernel oil fatty acid amidopropyl betaine, and the like, and alkyldimethylaminoacetic acid betaines such as lauryl dimethylaminoacetic acid betaine, coconut oil alkyldimethylaminoacetic acid betaine, and the like.

Examples of sulfobetaine-type amphoteric surfactants include lauryl hydroxysulfobetaine, lauric acid amidopropyl hydroxysulfobetaine, fatty acid amidopropyl hydroxysulfobetaine, and the like.

Examples of amidoamine oxide-type amphoteric surfactants include lauric acid amidopropyl dimethylamine oxide and the like.

In the cleaning agent of the present invention, one kind selected from the group consisting of the above-mentioned hydroxypropyl starch phosphate, fatty acid alkanolamide, glycine-type amphoteric surfactant, aminoacetic acid betaine-type amphoteric surfactant, sulfobetaine-type amphoteric surfactant, and amidoamine oxide-type amphoteric surfactant may be used as a thickener alone or two or more kinds thereof may also be selected and used in combination.

From the viewpoint of the thickening effect in the cleaning agent of the present invention, hydroxypropyl starch phosphate, fatty acid alkanolamide, and fatty acid amidopropyl betaine are preferably used, and hydroxypropyl starch phosphate, coconut oil fatty acid monoethanolamide, and coconut oil fatty acid amidopropyl betaine are more preferably used.

In one embodiment of the present invention, a cleaning agent can be provided that contains the cleansing composition of the present invention as a cleaning agent component and further contains a thickener.

The content of the cleansing composition of the present invention in the cleaning agent of this embodiment of the present invention is preferably 1 wt% to 50 wt%, more preferably 1 wt% to 30 wt%, further preferably 10 wt% to 30 wt%, as a total amount of the above-mentioned component (A), component (B), and component (C), or as a total amount of the above-mentioned component (A), component (B), component (C), and component (D).

In the cleaning agent of this embodiment of the present invention, the thickener to be used is preferably one or more kinds selected from the group consisting of the above-mentioned hydroxypropyl starch phosphate, fatty acid alkanolamide, glycine-type amphoteric surfactant, aminoacetic acid betaine-type amphoteric surfactant, sulfobetaine-type amphoteric surfactant, and amidoamine oxide-type amphoteric surfactant. The content of the thickener in the cleaning agent of this embodiment of the present invention is preferably 1 wt% to 5 wt%, more preferably 2 wt% to 5 wt%.

In another embodiment of the present invention, a cleaning agent can be provided that contains the cleansing composition of the present invention as a cleaning aid and further contains a thickener.

The content of the cleansing composition of the present invention in the cleaning agent of this embodiment of the present invention is preferably 0.5 wt% to 10 wt%, more preferably 1 wt% to 7 wt%, as a total amount of the above-mentioned component (A), component (B), and component (C), or as a total amount of the above-mentioned component (A), component (B), component (C), and component (D).

In the cleaning agent of this embodiment of the present invention, also, the thickener to be used is preferably one or more kinds selected from the group consisting of the above-mentioned hydroxypropyl starch phosphate, fatty acid alkanolamide, glycine-type amphoteric surfactant, aminoacetic acid betaine-type amphoteric surfactant, sulfobetaine-type amphoteric surfactant, and amidoamine oxide-type amphoteric surfactant. The content of such thickener is also the same as that in the cleaning agent of the above-mentioned embodiment.

The viscosity of the cleaning agent of each embodiment described above, that is, a cleaning agent containing the cleansing composition of the present invention as a cleaning agent component or cleaning aid and further containing a thickener, can be adjusted to fall within an appropriate range according to the form thereof, by appropriately setting the content of the cleansing composition of the present invention or the kind and content of the thickener, or by further combining the cleansing composition with other cleaning agent component, cleaning aid, and general additive.

In the cleaning agent of the above-mentioned embodiment of the present invention, the viscosity measured at 25°C using a B type viscometer can be adjusted within the range of preferably 500 mPa·s to 400,000 mPa·s, and the cleaning agent can be in the form of a liquid, paste, cream, or the like. In the cleaning agent of the above-mentioned embodiment of the present invention, the viscosity is preferably 500 mPa·s or more, 1,000 mPa·s or more, 2,000 mPa·s or more, 10,000 mPa·s or more, 30,000 mPa·s or more, or 100,000 mPa·s or more, as measured using a B type viscometer at 25°C, and may also be 1,000 mPa·s to 60,000 mPa·s, 2,000 mPa·s to 60,000 mPa·s, 10,000 mPa·s to 60,000 mPa·s, 30,000 mPa·s to 400,000 mPa·s, or 100,000 mPa·s to 300,000 mPa·s.

For example, in a cleaning agent containing the cleansing composition of the present invention as a cleaning agent component or cleaning aid, and further containing fatty acid alkanol amide and fatty acid amidopropylbetaine as thickeners, the viscosity can be increased to 500 mPa·s or more as measured using a B type viscometer at 25°C. In such cleaning agent, the viscosity is preferably 1,000 mPa·s to 60,000 mPa·s, more preferably 2,000 mPa·s to 60,000 mPa·s, further preferably 10,000 mPa·s to 60,000 mPa·s, as measured using a B type viscometer at 25°C. The cleaning agent of such embodiment of the present invention is in a viscous liquid to cream form and can be preferably provided as a hair shampoo, body shampoo, or the like.

In addition, for example, in a cleaning agent containing the cleansing composition of the present invention as a cleaning agent component or cleaning aid, and further containing hydroxypropyl starch phosphate as a thickener, the viscosity is preferably 30,000 mPa·s to 400,000 mPa·s, more preferably 100,000 mPa·s to 300,000 mPa·s, as measured using a B type viscometer at 25°C. The cleaning agent of such embodiment of the present invention is in a paste to cream form and can be preferably provided as a facial cleansing foam, facial cleansing cream, cleansing cream, or the like.

### [Example]

The present invention is described in more detail in the following with reference to Examples.

### [Examples 1 to 3, Reference Examples 1 to 3] Production of cleansing composition

L-alanine (50 g, 0.56 moles), decanoic acid (capric acid) (9 g, 0.05 moles), dodecanoic acid (lauric acid) (76 g, 0.38 moles), tetradecanoic acid (myristic acid) (15 g, 0.07 moles), and sodium hydroxide (18 g, 0.45 moles) were mixed and reacted for 23 hr under a nitrogen gas atmosphere at each temperature of 162°C, 170°C, or 147°C, while removing the generated water. The reaction mixture was cooled to give the cleansing compositions of Examples 1 to 3.

Decanoic acid, dodecanoic acid, and tetradecanoic acid were each used alone as the fatty acid, and the reaction was performed in the same manner as above at a reaction temperature of 162°C. The obtained reaction mixture was cooled to give the cleansing compositions of Reference Examples 1 to 3.

Commercially available products of L-alanine, decanoic acid, dodecanoic acid, tetradecanoic acid, and sodium hydroxide were used. Specifically, L-alanine was a product provided by Anhui Huaheng Biotechnology Co., Ltd., decanoic acid, dodecanoic acid, and tetradecanoic acid were products provided by FUJIFILM Wako Pure Chemical Corporation, and sodium hydroxide was a product provided by Showa Denko K.K.

The appearance of each of the cleansing compositions produced in the above-mentioned Examples 1 to 3 and Reference Examples 1 to 3 was observed when diluted with water to a solid content of 30 wt%.

Then, for the cleansing compositions produced in Examples 1 to 3 and Reference Example 1 that were observed to have a liquid appearance, the components contained in the compositions were analyzed by high-performance liquid chromatography (HPLC) method (HPLC: "Chromaster", Hitachi High-Tech Corporation; column: reverse-phase column (C18 column) "Inertsil ODS-3, particle size = 5 µm, 4.6 mm x 250 mm", GL Sciences Inc.).

Shampoos containing each of the cleansing compositions produced in Examples 1 to 3 and Reference Example 1 were prepared according to the formulations shown in Table 5 below, and the foaming ability and foam quality (foam fineness) were evaluated as follows. The content of each of the cleansing compositions produced in Examples 1 to 3 and Reference Example 1 was 30 wt % in a solid content.

### (1) Foaming ability

Shampoos containing respective cleansing compositions prepared above were diluted with water such that the content of the cleaning agent component was 1.5% by weight in a solid content, then stirred using a dynamic foam analyzer ("DFA100", KRUESS) for 20 seconds at 25°C. After 60 seconds, the foam volume was measured and evaluated according to the following criteria.

### <evaluation criteria>

A; 55 mL or more
B; 40 mL or more and less than 55 mL
C; less than 40 mL

### (2) Fineness of foam

In the same manner as in the above-mentioned evaluation of foaming ability, shampoos containing each cleansing composition were diluted with water such that the content of the cleaning agent component was 1.5 wt% in a solid content, then stirred using a dynamic foam analyzer. After 10 seconds, the particle size of the foam was calculated by image analysis of two-dimensional images and evaluated according to the following criteria.

### <evaluation criteria>

A; 2,250 µm² or less
B; more than 2,250 µm² and 3,500 µm² or less
C; more than 3,500 µm²

The results of the above-mentioned observation of the appearance of the cleansing compositions produced in Examples 1 to 3 and Reference Examples 1 and 3, as well as the results of the above-mentioned analysis and evaluation of the foaming ability and foam quality of the cleansing compositions produced in Examples 1 to 3 and Reference Example 1 are shown in Table 1.

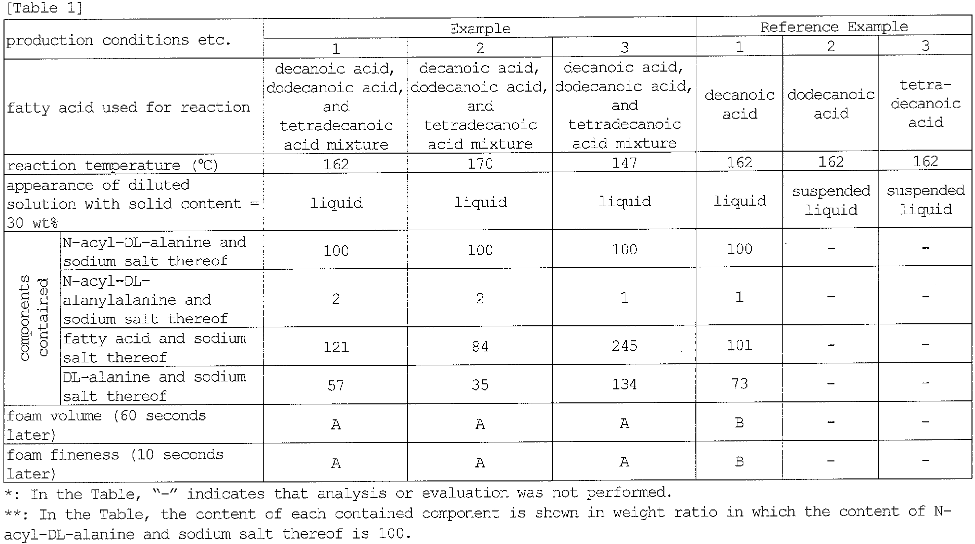

As shown in Table 1, when a mixture of decanoic acid, dodecanoic acid, and tetradecanoic acid, which have different carbon chain lengths, was used as the fatty acid to be reacted with L-alanine (Examples 1 to 3), the obtained cleansing composition had good solubility in water, and the shampoo containing the obtained cleansing composition exhibited good foaming ability, where the foam formed had good quality and formation of fine foam was observed.

On the other hand, when dodecanoic acid or tetradecanoic acid was used alone as the fatty acid to be reacted with L-alanine (Reference Examples 2 and 3), the obtained cleansing composition was inferior in solubility in water, whereas when decanoic acid was used (Reference Example 1), the obtained cleansing composition was soluble in water but the shampoo containing the aforementioned cleansing composition was inferior in foaming ability and foam quality.

From the above-mentioned evaluation results, therefore, it is suggested that the cleansing composition of the present invention preferably contains a reaction mixture obtained by reacting a mixture of two or more fatty acids having different carbon chain lengths with L-alanine in the presence of a basic substance, and further, that the cleansing composition is preferably a composition containing N-acyl-DL-alanine and a salt thereof, N-acyl-DL-alanylalanine and a salt thereof, fatty acid and a salt thereof, and unacylated DL-alanine and a salt thereof, obtained by reacting the aforementioned mixture of fatty acids with L-alanine at 147°C to 170°C in the presence of a basic substance, from the viewpoint of foaming ability and foam quality.

Then, the low temperature stability of the cleansing compositions produced in the above-mentioned Examples 1 to 3 was evaluated. Each cleansing composition of Examples 1 to 3 was diluted by adding water to a solid content of 30 wt%.

Each diluted solution (5 g) was dispensed into a 10 mL glass vial and placed in an incubator at 0°C. After 24 hr, the vial was removed and the appearance thereof was observed and evaluated according to the following criteria.

### <evaluation criteria>

A; transparent, liquid appearance, liquid appearance, no change in appearance observed before storage at 0°C
B: changes in appearance, such as cloudiness, solid precipitation, and the like were observed after storage at 0°C

The evaluation results of the above-mentioned low temperature stability of the cleansing compositions produced in Examples 1 to 3 are shown in Table 2.

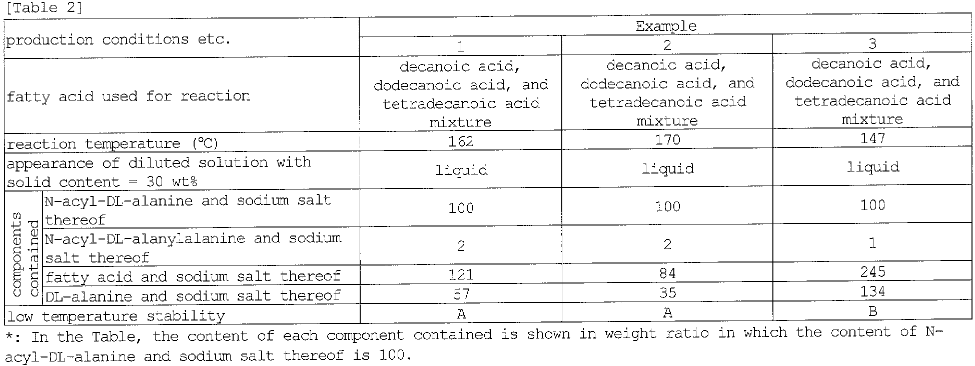

As shown in Table 2, when L-alanine, fatty acid, and sodium hydroxide were respectively reacted at 162°C and 170°C, no change in appearance of the obtained cleansing compositions (Examples 1 and 2) was observed after storage at 0°C for 24 hr.

On the other hand, when L-alanine, fatty acid, and sodium hydroxide were reacted at 147°C, the obtained cleansing composition (Example 3) became cloudy when stored at 0°C for 24 hr due to precipitation of fatty acid.

### [Examples 4 to 8, Comparative Examples 1 to 4] Cleansing composition

The cleansing composition of Comparative Example 1 was prepared by reacting L-alanine with coconut oil fatty acid chloride by a conventional method (Schotten-Baumann method). Furthermore, L-alanine (Junsei Chemical Co., Ltd.) and coconut oil fatty acid (NOF CORPORATION) were added to the cleansing composition of Comparative Example 1 to prepare the cleansing compositions of Comparative Examples 2 to 4 with the compositions shown in Table 3.

In addition, the cleansing composition produced in the above-mentioned Example 1 and the cleansing composition of Comparative Example 1 were mixed, and coconut oil fatty acid (NOF CORPORATION) and L-alanine (Junsei Chemical Co., Ltd.) were added to prepare the cleansing compositions of Examples 4 and 7 with the compositions shown in Table 3. Furthermore, coconut oil fatty acid (NOF CORPORATION) or L-alanine (Junsei Chemical Co., Ltd.) was added to the cleansing composition produced in the above-mentioned Example 1 to prepare the cleansing composition of Example 5 or 6 with the compositions shown in Table 3. Furthermore, coconut oil fatty acid (NOF CORPORATION) and L-alanine (Junsei Chemical Co., Ltd.) were added to the cleansing composition of Comparative Example 1 to prepare the cleansing composition of Example 8 with the composition shown in Table 3.

Each of the cleansing compositions produced in Examples 1 to 3, and each of the cleansing compositions of the above-mentioned Examples 4 to 8 and Comparative Examples 1 to 4, was dissolved in water to a solid content of 20 wt% to prepare an aqueous solution, which was then further diluted with water to a solid content of 1.5 wt%.

### (1) Foaming ability

Diluted solutions of the aqueous solutions of each cleansing composition prepared above were stirred using a dynamic foam analyzer ("DFA100", KRUESS) for 20 seconds at 25°C. After 30 seconds, the foam volume was measured and evaluated according to the following criteria.

### <evaluation criteria>

AA; 50 mL or more
A; 40 mL or more and less than 50 mL
B; 35 mL or more and less than 40 mL
C; less than 35 mL

### (2) Fineness of foam

Diluted solutions of the aqueous solutions of each cleansing composition prepared above were stirred using a dynamic foam analyzer for 20 seconds at 25°C. After 30 seconds, the particle size of the foam was calculated by image analysis of two-dimensional images and evaluated according to the following criteria.

### <evaluation criteria>

AA; 5,500 µm² or less
A; more than 5,500 µm² and 6,500 µm² or less
B; more than 6,500 µm² and 7,000 µm² or less
C; more than 7,000 µm²

The evaluation results of foaming ability and fineness of foam are shown together in Table 3.

**[Table 3]**

| component | Example | | | | | | | | Comparative Example | | | |
|---|---|---|---|---|---|---|---|---|---|---|---|---|
| | 1 | 2 | 3 | 4 | 5 | 6 | 7 | 8 | 1 | 2 | 3 | 4 |
| N-acyl-DL-alanine and sodium salt thereof | 100 | 100 | 100 | 100 | 100 | 100 | 100 | 100 | 100 | 100 | 100 | 100 |
| N-acyl-DL-alanylalanine and sodium salt thereof | 2 | 2 | 1 | 3 | 2 | 2 | 3 | 3 | 3 | 3 | 3 | 3 |
| fatty acid and sodium salt thereof | 121 | 84 | 245 | 54 | 183 | 121 | 97 | 47 | 6 | 6 | 15 | 11 |
| L-alanine, DL-alanine, and sodium salts thereof | 57 | 35 | 134 | 118 | 57 | 103 | 10 | 134 | 0 | 144 | 191 | 211 |
| foam volume (30 seconds later) | A | A | AA | A | AA | A | AA | A | C | C | A | B |
| foam fineness (30 seconds later) | AA | A | A | AA | AA | A | AA | AA | C | C | B | A |

| | | | | | | | | | | | | |
|---|---|---|---|---|---|---|---|---|---|---|---|---|
| *: In the Table, the content of each component contained is shown in weight ratio in which the content of N-acyl-DL-alanine and sodium salt thereof is 100. | | | | | | | | | | | | |

As shown in Table 3, the aqueous solutions of the cleansing compositions produced in the above-mentioned Examples 1 to 3 and the diluted solutions of the aqueous solutions of the cleansing compositions of Examples 4 to 8 obtained "A" or higher evaluation for both foaming ability and foam fineness.

On the other hand, the diluted solutions of each aqueous solution of the cleansing composition of Comparative Example 1, which did not contain unacylated L-alanine, DL-alanine, or sodium salts thereof and in which the content of fatty acids and sodium salts thereof was less than 45 parts by weight per 100 parts by weight of N-acyl-DL-alanine and sodium salts thereof, and the cleansing composition of Comparative Example 2, in which the content of fatty acids and sodium salts thereof was less than 45 parts by weight per 100 parts by weight of N-acyl-DL-alanine and sodium salts thereof, were rated "C" for both foaming ability and foam quality (foam fineness). Furthermore, in the diluted solutions of each aqueous solution of the cleansing compositions of Comparative Examples 3 and 4, in which the content of fatty acids and sodium salts thereof was less than 45 parts by weight per 100 parts by weight of N-acyl-DL-alanine and sodium salt thereof, and the content of unacylated L-alanine, DL-alanine, and sodium salts thereof was far greater than 150 parts by weight per 100 parts by weight of N-acyl-DL-alanine and sodium salt thereof, the former received a rating of "B" for foam quality (foam fineness), and the latter received a rating of "B" for foaming ability.

Then, the cleansing composition produced in the above-mentioned Example 1 and the cleansing compositions of the above-mentioned Examples 4, 6, and 8 were evaluated for feeling of use. At the same time, evaluations were simultaneously conducted for the cleansing compositions of Comparative Example 5, in which coconut oil fatty acid (NOF CORPORATION) was added to the cleansing composition of Comparative Example 1 to prepare the composition shown in Table 4, and Comparative Example 6, in which coconut oil fatty acid (NOF CORPORATION) and L-alanine (Junsei Kagaku Co., Ltd.) were added to the cleansing composition of Comparative Example 1 to prepare the composition shown in Table 4.

For evaluation of the feeling of use, five panelists washed their skin and hair with an aqueous solution prepared by dissolving each cleansing composition in water to a solid content of 20 wt%, and performed sensory evaluations of the foaming, creaminess of the foam, moist feeling of finish, smoothness when applied to skin, and combability when applied to hair, according to the following evaluation criteria. The average score of the five panelists was calculated for each evaluation item, and the results, judged according to the following criteria, are shown together in Table 4.

### <evaluation criteria>

Very good: 5 points
Good: 4 points
Average: 3 points
Slightly poor: 2 points
Poor: 1 point

### <judgment criteria>

AA: 3.5 points or higher
A: 3 points or higher and less than 3.5 points
B: 2 points or higher and less than 3 points
C: less than 2 points

**[Table 4]**

| component | Example | | | | Comparative Example | |
|---|---|---|---|---|---|---|
| | 1 | 4 | 6 | 8 | 5 | 6 |
| N-acyl-DL-alanine and sodium salt thereof | 100 | 100 | 100 | 100 | 100 | 100 |
| N-acyl-DL-alanylalanine and sodium salt thereof | 2 | 3 | 2 | 3 | 3 | 3 |
| fatty acid and sodium salt thereof | 121 | 54 | 121 | 47 | 479 | 310 |
| L-alanine,DL-alanine and sodium salts thereof | 57 | 118 | 103 | 134 | 0 | 2 |
| foaming | AA | A | AA | A | C | C |
| creaminess of foam | AA | AA | AA | AA | A | B |
| moist feeling of finish | AA | A | AA | A | B | A |
| smoothness when applied to skin | AA | AA | A | AA | B | B |
| combability when applied to hair | AA | AA | AA | AA | B | B |

| | | | | | | |
|---|---|---|---|---|---|---|
| *: In the Table, the content of each component contained is shown in weight ratio in which the content of N-acyl-DL-alanine and sodium salt thereof is 100. | | | | | | |

As shown in Table 4, the cleansing composition produced in Example 1 and the cleansing compositions of Examples 4, 6, and 8 were rated "AA" or "A" in all items of foaming, creamy foam, moist feeling of finish, smoothness upon application to skin, and combability upon application to hair, when an aqueous solution of each cleansing composition was used.

On the other hand, the cleansing compositions of Comparative Examples 5 and 6, which either did not contain L-alanine not N-acylated, DL-alanine not N-acylated, or sodium salts thereof, or contained less than 10 parts by weight thereof per 100 parts by weight of N-acyl-DL-alanine and sodium salts thereof, and contained far more than 245 parts by weight of fatty acids and sodium salts thereof per 100 parts by weight of N-acyl-DL-alanine and sodium salts thereof, received "C" for foaming when aqueous solutions thereof were used. The creaminess of the foam when the aqueous solution of the cleansing composition of Comparative Example 5 was used, and the moist feeling of finish when the cleansing composition of Comparative Example 6 was used, were both rated "A", but the feeling of use when the aqueous solution of each cleansing composition was used was always rated "B."

The above-mentioned evaluation results of the foaming, foam quality, and feeling of use of the cleansing compositions produced in Examples 1 to 3, the cleansing compositions of Examples 4 to 8, and the cleansing compositions of Comparative Examples 1 to 6 show that the cleansing compositions of the present invention containing components (A) through (D) are superior in foaming and foam quality, as well as feeling of use when used to wash skin and hair.

Furthermore, it was suggested that good foaming ability and foam quality can be achieved when the contents of component (B) and component (C) in the cleansing composition are 45 to 245 parts by weight and 10 to 150 parts by weight, respectively, per 100 parts by weight of component (A).

### [Example 9, Comparative Examples 7 to 12] Shampoo

The components listed in Table 5 were added sequentially to water, mixed homogeneously, and dissolved to prepare the shampoos of Example 9 and Comparative Examples 7 to 12.

For the shampoos of Example 9 and Comparative Examples 7 to 12, pH was measured at 25°C by the glass electrode method, and evaluation of the foam quality (foam fineness), measurement of the coefficient of friction of the hair bundle after washing, and evaluation of the feel of the hair bundle after washing were performed as follows. The measurement and evaluation results are shown together in Table 5.

### (1) Evaluation of foam quality (foam fineness)

Each shampoo of the Examples and Comparative Examples was diluted with hard water (hardness = 300 ppm) such that the content of cleaning agent components was 1.5 wt% in solid content. The diluted shampoo was then stirred for 20 seconds at 25°C using a dynamic foam analyzer (DFA100, KRUESS). The particle size of the foam after 10 seconds and 180 seconds was calculated by image analysis of two-dimensional images and evaluated according to the following criteria.

### <evaluation criteria for foam fineness after 10 seconds>

AA: 2,500 µm² or less
A: more than 2,500 µm² and less than 4,000 µm²
B: more than 4,000 µm² and less than 5,000 µm²

### C: more than 5,000 µm²

### <evaluation criteria for foam fineness after 180 seconds>

AA: 7,500 µm² or less
A: more than 7,500 µm² and less than 10,000 µm²
B: more than 10,000 µm² and less than 12,500 µm²
C: more than 12,500 µm²

### (2) Measurement of coefficient of friction in hair bundle after washing

Each shampoo of the Examples and Comparative Examples was diluted 7 times with hard water (hardness = 300 ppm), and hair bundle (10 g, 30 cm) was immersed in 98 g of the aforementioned diluted solution at 25°C for 5 min. The hair bundle was further immersed in 800 g of hard water (hardness = 300 ppm) for 30 seconds, and this was repeated twice. The hair bundle was then towel-dried for 10 seconds, and the coefficient of friction of the hair bundle was measured three times using a friction measurement device ("TL201Ts", Trinity Lab Co., Ltd.). The average measurement value was calculated and evaluated according to the following evaluation criteria.

### <evaluation criteria>

AA; coefficient of friction is 0.225 or less
A: coefficient of friction is more than 0.225 and 0.25 or less
B: coefficient of friction is more than 0.25 and 0.275 or less
C: coefficient of friction is more than 0.275

### (3) Evaluation of feel of hair bundle after washing

Each shampoo of the Examples and Comparative Examples was diluted 7 times with hard water (hardness = 300 ppm), and hair bundle (10 g, 30 cm) was immersed in 98 g of the aforementioned diluted solution at 25°C for 5 min. The hair bundle was then immersed in 800 g of hard water (300 ppm) for 30 seconds, and this was repeated twice. The hair bundle was then dried in a constant temperature room (25°C). One panelist evaluated the feel of the hair bundle after drying according to the following evaluation criteria.

### <evaluation criteria>

Very good: AA
Good: A
Average: B
Poor: C

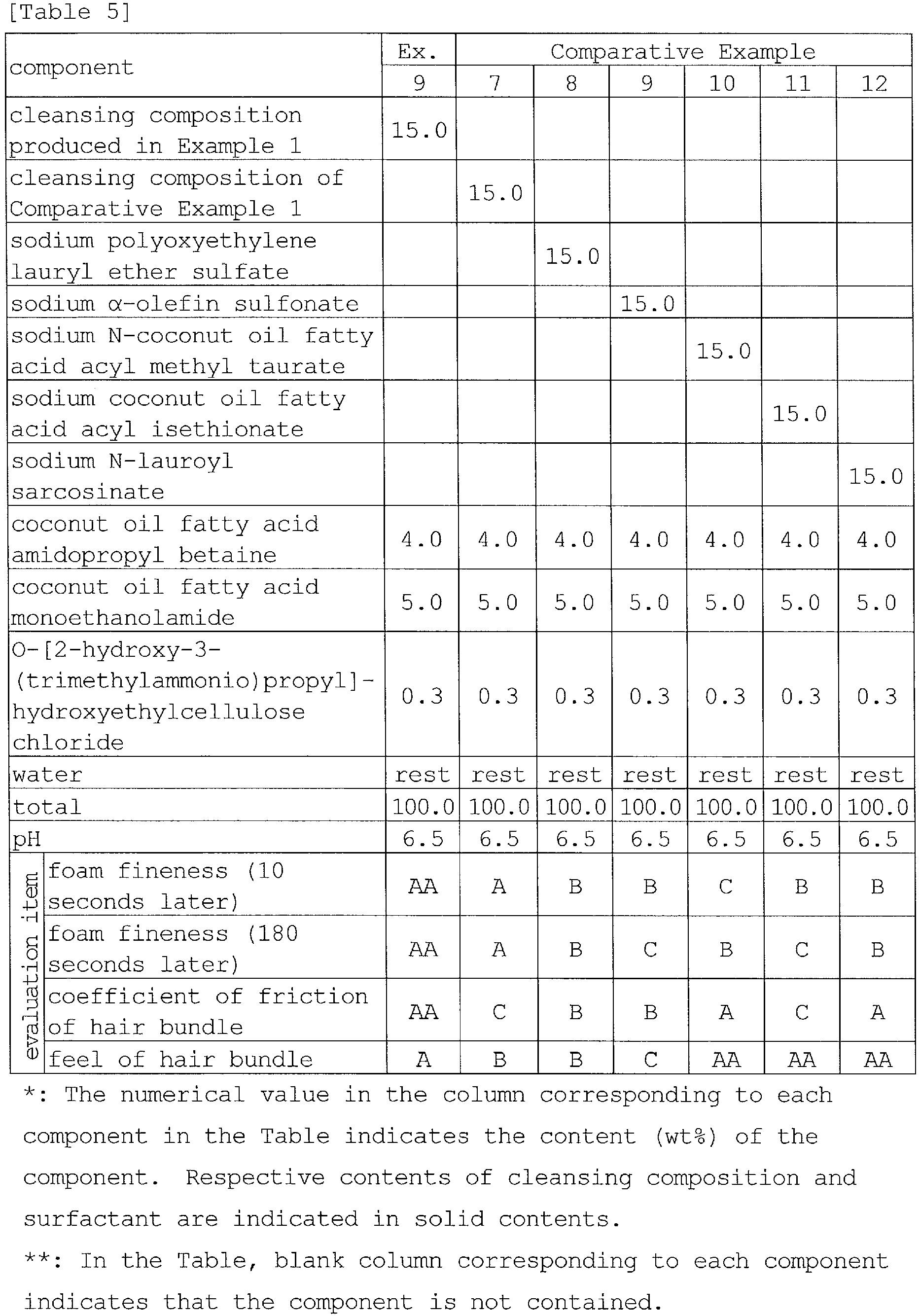

As shown in Table 5, the shampoo of Example 9, which contained the cleansing composition produced in the above-mentioned Example 1 as a cleaning agent component, was superior in foam quality and smoothness, which is shown by the coefficient of friction after washing hair bundle, and affords a favorable feel of the hair bundle, compared to respective shampoos of Comparative Examples 7 to 12, which used the cleansing composition of Comparative Example 1 and commercially available anionic surfactants (sodium polyoxyethylene lauryl ether sulfate, sodium α-olefin sulfonate, sodium N-coconut oil fatty acid acyl methyl taurate, sodium coconut oil fatty acid acyl isethionate, sodium N-lauroyl sarcosinate) as cleaning agent components.

The above-mentioned evaluation results suggest that cleaning agents containing the cleansing composition of the present invention as a cleaning agent component produce good foam quality, can provide a smooth and pleasant feel to hair, and can improve the frictional feeling after washing.

### [Examples 10 to 14, Comparative Examples 13 to 17] Shampoo

In the shampoo of Example 9 shown in Table 5, the cleansing composition produced in Example 1 was replaced with 10 wt% (solid content) of the cleaning agent components shown in Table 6 (sodium polyoxyethylene lauryl ether sulfate, sodium N-coconut oil fatty acid acyl methyl taurate, sodium N-lauroyl sarcosinate, sodium α-olefin sulfonate, and alkyl polyglucoside), and 5 wt% (solid content) of the cleansing composition produced in Example 1 or Comparative Example 1 as a cleaning aid to prepare shampoos. These shampoos were used as the shampoos of Examples 10 to 14 and Comparative Examples 13 to 17, respectively.

The viscosity of each shampoo of Examples 10 to 14 and Comparative Examples 13 to 17 was measured at 25°C using a B type viscometer and evaluated according to the following criteria. The evaluation results are shown in Table 6.

### <evaluation criteria>

AA; viscosity is 10,000 mPa·s or more
A; viscosity is 2,000 mPa·s or more and less than 10,000 mPa·s
B; viscosity is 1,000 mPa·s or more and less than 2,000 mPa·s
C; viscosity is 500 mPa·s or more and less than 1,000 mPa·s
D; viscosity is less than 500 mPa·s

**[Table 6]**

| sample | cleaning agent component | cleaning aid | viscosity |
|---|---|---|---|
| Example 10 | sodium polyoxyethylene lauryl ether sulfate | cleansing composition produced in Example 1 | AA |
| Example 11 | sodium N-coconut oil fatty acid acyl methyl taurate | cleansing composition produced in Example 1 | A |
| Example 12 | sodium N-lauroyl sarcosinate | cleansing composition produced in Example 1 | A |
| Example 13 | sodium α-olefin sulfonate | cleansing composition produced in Example 1 | AA |
| Example 14 | alkyl polyglucoside | cleansing composition produced in Example 1 | B |
| Comparative Example 13 | sodium polyoxyethylene lauryl ether sulfate | cleansing composition of Comparative Example 1 | D |
| Comparative Example 14 | sodium N-coconut oil fatty acid acyl methyl taurate | cleansing composition of Comparative Example 1 | D |
| Comparative Example 15 | sodium N-lauroyl sarcosinate | cleansing composition of Comparative Example 1 | D |
| Comparative Example 16 | sodium α-olefin sulfonate | cleansing composition of Comparative Example 1 | C |
| Comparative Example 17 | alkyl polyglucoside | cleansing composition of Comparative Example 1 | D |

As shown in Table 6, the shampoos of Examples 10 to 14, which contained each of sodium polyoxyethylene lauryl ether sulfate, sodium N-coconut oil fatty acid acyl methyl taurate, sodium N-lauroyl sarcosine, sodium α-olefin sulfonate, and alkyl polyglucoside as the cleaning agent component, the cleansing composition produced in Example 1 as a cleaning aid, and coconut oil fatty acid monoethanolamide and coconut oil fatty acid amidopropyl betaine as thickeners, all showed a good thickening of 1,000 mPa·s or more, thus demonstrating superior handleability as cleaning agents.

On the other hand, the shampoos of Comparative Examples 13 to 17, which contained coconut oil fatty acid monoethanolamide and coconut oil fatty acid amidopropyl betaine as thickeners and the cleansing composition of Comparative Example 1 as a cleaning aid, did not show thickening of 500 mPa·s or more, except for the shampoo of Comparative Example 16. The viscosity of the shampoo of Comparative Example 16 was also less than 1,000 mPa·s.

Other examples of the cleaning agent of the present invention are successively shown below. The pH of the cleaning agents in each of the following examples was measured using the glass electrode method in accordance with General Test Method 2.54, pH Measurement Method, of the Japanese Pharmacopoeia, 18th Edition.

### [Example 15] Shampoo

In Table 7, (1) to (8) were added sequentially to (10), mixed, and dissolved homogeneously, and the pH was adjusted to 6.5 with (9).

**[Table 7]**

| component | | content (wt%) |
|---|---|---|
| (1) | cleansing composition produced in Example 1 | 15.0 |
| (2) | coconut oil fatty acid monoethanolamide | 5.0 |
| (3) | coconut oil fatty acid amidopropyl betaine | 4.0 |
| (4) | glycerin | 1.0 |
| (5) | sodium chloride | 1.0 |
| (6) | O-[2-hydroxy-3-(trimethylammonio)propyll-hydroxyethylcellulose chloride | 0.3 |
| (7) | preservative | 0.1 |
| (8) | fragrance | 0.1 |
| (9) | citric acid | appropriate amount |
| (10) | water | rest |
| total | | 100 |

| | | |
|---|---|---|
| *: In the Table, the contents of (1) to (3) are indicated in solid contents. | | |

### [Example 16] Shampoo

In Table 8, (1) to (7) were added sequentially to (9), mixed, and dissolved homogeneously, and the pH was adjusted to 7.0 with (8).

**[Table 8]**

| component | | content (wt%) |
|---|---|---|
| (1) | cleansing composition produced in Example 1 | 10.0 |
| (2) | decyl glucoside | 10.0 |
| (3) | 3-[(dodecanamidomethyl)dimethylammonio]-2-hydroxypropane-1-sulfonate | 5.0 |
| (4) | 1,3-butylene glycol | 2.0 |
| (5) | O-[2-hydroxy-3-(trimethylammonio)propyl]guar gum chloride | 0.2 |
| (6) | preservative | 0.1 |
| (7) | fragrance | 0.1 |
| (8) | citric acid | appropriate amount |
| (9) | water | rest |
| total | | 100 |

| | | |
|---|---|---|
| *: In the Table, the contents of (1) to (3) are indicated in solid contents. | | |

### [Example 17] Shampoo

In Table 9, (1) to (7) were added sequentially to (9), mixed, and dissolved homogeneously, and the pH was adjusted to 7.0 with (8).

**[Table 9]**

| component | | content (wt%) |
|---|---|---|
| (1) | cleansing composition produced in Example 1 | 5.0 |
| (2) | sodium polyoxyethylene lauryl ether sulfate | 15.0 |
| (3) | coconut oil fatty acid diethanolamide | 5.0 |
| (4) | zinc L-pyrrolidone carboxylate | 0.3 |
| (5) | N-coconut oil fatty acid acyl-L-arginine ethyl ester-DL-pyrrolidone carboxylate | 0.1 |
| (6) | preservative | 0.1 |
| (7) | fragrance | 0.1 |
| (8) | citric acid | appropriate amount |
| (9) | water | rest |
| total | | 100 |

| | | |
|---|---|---|
| *: In the Table, the contents of (1) to (3) and (5) are indicated in solid contents. | | |

### [Example 18] Body shampoo

In Table 10, (1) to (8) were added sequentially to (10), mixed, and dissolved homogeneously, and the pH was adjusted to 7.0 with (9).

**[Table 10]**

| component | | content (wt%) |
|---|---|---|
| (1) | cleansing composition produced in Example 1 | 15.0 |
| (2) | coconut oil alkyl glucoside | 10.0 |
| (3) | coconut oil fatty acid diethanolamide | 5.0 |
| (4) | sodium chloride | 1.0 |
| (5) | magnesium chloride | 1.0 |
| (6) | glycerin | 5.0 |
| (7) | preservative | 0.1 |
| (8) | fragrance | 0.1 |
| (9) | citric acid | appropriate amount |
| (10) | water | rest |
| total | | 100 |

| | | |
|---|---|---|
| *: In the Table, the contents of (1) to (3) are indicated in solid contents. | | |

### [Example 19] Facial cleansing foam

In Table 11, (1) to (7) were added sequentially to (9), mixed, and dissolved homogeneously, and the pH was adjusted to 6.5 with (8).

**[Table 11]**

| component | | content (wt%) |
|---|---|---|
| (1) | cleansing composition produced in Example 1 | 10.0 |
| (2) | decyl glucoside | 10.0 |
| (3) | 3-[(dodecanamidomethyl)dimethylammonio]-2-hydroxypropane-1-sulfonate | 3.0 |
| (4) | glycerin | 1.0 |
| (5) | O-[2-hydroxy-3-(trimethylammonio)propyl]guar gum chloride | 0.2 |
| (6) | preservative | 0.1 |
| (7) | fragrance | 0.1 |
| (8) | citric acid | appropriate amount |
| (9) | water | rest |
| total | | 100 |

| | | |
|---|---|---|
| *: In the Table, the contents of (1) to (3) are indicated in solid contents. | | |

### [Example 20] Facial cleansing foam

In Table 12, (1) to (13) were added to 30 g of (15), dispersed, and dissolved by heating to 70°C to 80°C. Separately, (14) was dissolved in the remaining (15) and gradually added while stirring. After the saponification reaction was completed, the mixture was cooled to room temperature.

**[Table 12]**

| component | | content (wt%) |
|---|---|---|
| (1) | cleansing composition produced in Example 1 | 1.5 |
| (2) | lauric acid | 8.0 |
| (3) | myristic acid | 15.0 |
| (4) | stearic acid | 1.5 |
| (5) | palmitic acid | 1.5 |
| (6) | glycol stearate | 2.0 |
| (7) | kaolin | 0.5 |
| (8) | glycerin | 7.0 |
| (9) | polyethylene glycol 400 | 5.0 |
| (10) | coconut oil fatty acid amidopropyl betaine | 3.0 |
| (11) | zinc L-pyrrolidone carboxylate | 1.2 |
| (12) | preservative | 0.1 |
| (13) | fragrance | 0.1 |
| (14) | sodium hydroxide | 4.4 |
| (15) | water | rest |
| total | | 100 |

| | | |
|---|---|---|
| *: In the Table, the contents of (1) and (10) are indicated in solid contents. | | |

### [Examples 21 to 24, Comparative Examples 18, 19] Facial cleansing foam

In Table 13, (1) and (3) to (5) were added to (10) and dissolved by heating to 70°C to 80°C. Separately, (7) was dispersed in (6) heated to 80°C, added to the aforementioned solution while stirring and homogeneously mixed. (8) was added and the mixture was cooled to 45°C. (9) was then added and mixed to prepare the facial cleansing foams of Examples 21 to 24.

Furthermore, the facial cleansing foams of Comparative Examples 18 and 19 were prepared in the same manner except that (2) was used instead of (1) in Table 13.

The viscosity of each facial cleansing foam of Examples 21 to 24 and Comparative Examples 18 and 19 was measured at 25°C using a B type viscometer and evaluated according to the following criteria.

### <evaluation criteria>

A; viscosity is 100,000 mPa·s or more
B; viscosity is 30,000 mPa·s or more and less than 100,000 mPa·s
C; viscosity is less than 30,000 mPa·s

In addition, the appearance of each facial cleansing foam of Examples 21 to 24 and Comparative Examples 18 and 19 was observed at ordinary temperature and evaluated according to the following criteria.

### <evaluation criteria>

A; shows homogeneous white paste
B; shows homogeneous white liquid
C; separation into two layers of aqueous layer and oil layer is observed

The evaluation results are shown together in Table 13.

**[Table 13]**

| component | | Example | | | | Comparative Example | |
|---|---|---|---|---|---|---|---|
| | | 21 | 22 | 23 | 24 | 18 | 19 |
| (1) | cleansing composition produced in Example 1 | 12.3 | 12.3 | 12.3 | 12.3 | | |
| (2) | cleansing composition of Comparative Example 1 | | | | | 12.3 | 12.3 |
| (3) | N-lauroyl-N'-carboxymethyl-N'-hydroxyethyl-ethylenediamine sodium | 5.9 | 5.9 | 5.9 | 5.9 | 5.9 | 5.9 |
| (4) | glycerin | 5.0 | 5.0 | 5.0 | 5.0 | 5.0 | 5.0 |
| (5) | alkyl acrylate copolymer emulsion (30 wt% aqueous solution) | 1.0 | 1.0 | 1.0 | 1.0 | 1.0 | 1.0 |
| (6) | vaseline | 10.0 | 10.0 | 10.0 | 10.0 | 10.0 | 10.0 |
| (7) | hydroxypropyl starch phosphate | 2.0 | 2.5 | 3.0 | 4.0 | 2.0 | 3.0 |
| (8) | citric acid | 0.7 | 0.7 | 0.7 | 0.7 | 0.7 | 0.7 |
| (9) | preservative | 0.3 | 0.3 | 0.3 | 0.3 | 0.3 | 0.3 |
| (10) | water | rest | rest | rest | rest | rest | rest |
| total | | 100.0 | 100.0 | 100.0 | 100.0 | 100.0 | 100.0 |
| evaluation item | viscosity | B | B | A | A | C | C |
| | appearance | B | B | A | A | B | C |

| | | | | | | | |
|---|---|---|---|---|---|---|---|
| *: The numerical value in the column corresponding to each component in the Table indicates the content (wt%) of the component. Respective contents of cleansing composition, surfactant, and alkyl acrylate copolymer emulsion are indicated in solid contents. **: In the Table, blank columns corresponding to cleansing composition produced in Example 1 and cleansing composition of Comparative Example 1 indicates that the components are not contained. | | | | | | | |

In Table 13, the facial cleansing foams of Examples 21 to 24, which contained the cleansing composition produced in the above-mentioned Example 1 as a cleaning agent component, exhibited good thickening due to the addition of hydroxypropyl starch phosphate. The facial cleansing foams of Examples 23 and 24, in which hydroxypropyl starch phosphate was added in amounts of 3.0 wt% and 4.0 wt %, respectively, produced homogeneous, paste-like facial cleansing foams.

On the other hand, the facial cleansing foams of Comparative Examples 18 and 19 contained the cleansing composition of Comparative Example 1, which did not contain unacylated L-alanine, unacylated DL-alanine, or sodium salts thereof, and in which the content of fatty acids and sodium salts thereof was less than 45 parts by weight per 100 parts by weight of N-acyl-DL-alanine and sodium salts thereof. They did not show thickening due to the addition of hydroxypropyl starch phosphate, and a homogeneous paste-like facial cleansing foam was not obtained.

The above results suggest that good thickening can be achieved by adding hydroxypropyl starch phosphate to cleaning agent of the present invention that contains the cleansing composition of the present invention as a cleaning agent component.

### [Industrial Applicability]

As described in detail above, the present invention can provide a cleansing composition that is superior in foaming and foam quality, affords good feeling of use, can impart a moist feeling after washing and drying, and further improves a frictional feeling after washing. The cleansing composition of the present invention can be used as a cleaning agent component or a cleaning aid in cleaning agents for skin and hair.

In addition, the present invention can provide a method for producing a cleansing composition that imposes little burden on the environment and allows the reaction to proceed under relatively mild conditions. According to the production method of the present invention, the reaction mixture can be used as is as a cleansing composition.

Furthermore, the present invention can provide a cleaning agent containing the above-mentioned cleansing composition, that is superior in foaming and foam quality, can impart a good moist feeling to skin or hair after washing and drying, improves a frictional feeling after washing, and is superior in a feeling of use.

This application is based on a patent application No. 2023-156575 filed in Japan, the contents of which are incorporated in full herein.

## Claims

1. A cleansing composition comprising (A) one or more kinds selected from the group consisting of N-acyl alanine having an acyl group having 8 to 22 carbon atoms and a salt thereof, (B) one or more kinds selected from the group consisting of fatty acid having 8 to 22 carbon atoms and a salt thereof, and (C) one or more kinds selected from the group consisting of alanine and a salt thereof, and having a content ratio of the component (A), component (B), and component (C) [(A):(B):(C)] of 100:45 to 245:10 to 150 by weight.

2. The cleansing composition according to claim 1, further comprising (D) one or more kinds selected from the group consisting of N-acylalanylalanine having an acyl group having 8 to 22 carbon atoms and a salt thereof.

3. The cleansing composition according to claim 2, wherein a content ratio of component (D) to component (A) [(A):(D)] is 100:1 to 10 by weight.

4. The cleansing composition according to any one of claims 1 to 3, comprising two or more kinds of N-acyl alanines having an acyl group having a different carbon number or salts thereof as the (A) one or more kinds selected from the group consisting of N-acyl alanine having an acyl group having 8 to 22 carbon atoms and a salt thereof.

5. The cleansing composition according to any one of claims 1 to 3, comprising two or more kinds of fatty acids having a different carbon number or salts thereof as the (B) one or more kinds selected from the group consisting of fatty acid having 8 to 22 carbon atoms and a salt thereof.

6. The cleansing composition according to claim 2 or 3, comprising two or more kinds of N-acyl alanylalanines having an acyl group having a different carbon number or salts thereof as the (D) one or more kinds selected from the group consisting of N-acyl alanylalanine having an acyl group having 8 to 22 carbon atoms and a salt thereof.

7. A method for producing a cleansing composition, comprising reacting (a) fatty acid having 8 to 22 carbon atoms, (b) alanine, and (c) one or more kinds selected from the group consisting of alkali metal hydroxide and amine at 147°C to 190°C.

8. The production method according to claim 7, wherein the (a) fatty acid having 8 to 22 carbon atoms is used in 0.5 moles to 1.5 moles per mole of (b) alanine.

9. The production method according to claim 7 or 8, wherein the (c) one or more kinds selected from the group consisting of alkali metal hydroxide and amine is used in 0.5 moles to 1.5 moles per mole of (b) alanine.

10. The production method according to claim 7 or 8, wherein the (a) fatty acid having 8 to 22 carbon atoms is a mixture of two or more kinds of fatty acids having a different carbon number.

11. The production method according to claim 7 or 8, wherein the (b) alanine is L-alanine or DL-alanine.

12. The production method according to claim 7 or 8, wherein the (c) one or more kinds selected from the group consisting of alkali metal hydroxide and amine is sodium hydroxide.

13. A cleaning agent comprising the cleansing composition according to any one of claims 1 to 3 as a cleaning agent component.

14. The cleaning agent according to claim 13, having a viscosity of 500 mPa·s to 60,000 mPa·s as measured using a B type viscometer at 25°C.

15. A cleaning agent comprising the cleansing composition according to any one of claims 1 to 3 as a cleaning aid.

16. The cleaning agent according to claim 15, having a viscosity of 500 mPa·s to 60,000 mPa·s as measured using a B type viscometer at 25°C.

17. A cleaning agent comprising the cleansing composition according to any one of claims 1 to 3 as a cleaning agent component and further comprising a thickener.

18. The cleaning agent according to claim 17, wherein the thickener is one or more kinds selected from the group consisting of hydroxypropyl starch phosphate, fatty acid alkanolamide, glycine-type amphoteric surfactant, aminoacetic acid betaine-type amphoteric surfactant, sulfobetaine-type amphoteric surfactant, and amidoamine oxide-type amphoteric surfactant.

19. The cleaning agent according to claim 17, wherein a content of the thickener is 1 wt% to 5 wt% with respect to the total amount of the cleaning agent.

20. The cleaning agent according to claim 17, having a viscosity of 500 mPa·s to 400,000 mPa·s as measured using a B type viscometer at 25°C.

21. A cleaning agent comprising the cleansing composition according to any one of claims 1 to 3 as a cleaning aid and further comprising a thickener.

22. The cleaning agent according to claim 21, wherein the thickener is one or more kinds selected from the group consisting of hydroxypropyl starch phosphate, fatty acid alkanolamide, glycine-type amphoteric surfactant, aminoacetic acid betaine-type amphoteric surfactant, sulfobetaine-type amphoteric surfactant, and amidoamine oxide-type amphoteric surfactant.

23. The cleaning agent according to claim 21, wherein a content of the thickener is 1 wt% to 5 wt% with respect to the total amount of the cleaning agent.

24. The cleaning agent according to claim 21, having a viscosity of 500 mPa·s to 400,000 mPa·s as measured using a B type viscometer at 25°C.
